(19) Europäisches Patentamt
European Patent Office
Office européen des brevets

(11) EP 2 503 331 A2

(12) EUROPEAN PATENT APPLICATION
published in accordance with Art. 153(4) EPC

(43) Date of publication:
26.09.2012 Bulletin 2012/39

(51) Int Cl.:
G01N 33/12 (2006.01)   G06T 7/00 (2006.01)
A22C 25/00 (2006.01)   G06K 9/64 (2006.01)
G01N 21/88 (2006.01)   G06K 9/62 (2006.01)
G01N 21/95 (2006.01)

(21) Application number: 10829612.0

(22) Date of filing: 15.11.2010

(86) International application number:
PCT/IB2010/055172

(87) International publication number:
WO 2011/058529 (19.05.2011 Gazette 2011/20)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 16.11.2009 CL 20852009

(71) Applicant: Pontificia Universidad Catolica De
Chile
Santiago de Chile 8331150 (CL)

(72) Inventors:
• AGUILERA RADIC, José Miguel
Santiago 8331150 (CL)

• CIPRIANO ZAMORANO, Aldo Francisco José
Tomás
Santiago 8331150 (CL)
• MERY QUIROZ, Domingo Arturo
Santiago 8331150 (CL)
• SOTO ARRIAZA, Alvaro Marcelo Rodrigo
Santiago 8331150 (CL)

(74) Representative: Carvajal y Urquijo, Isabel et al
Clarke, Modet & Co.
c/ Goya, 11
28001 Madrid (ES)

(54) METHOD AND SYSTEM FOR THE REAL-TIME AUTOMATIC ANALYSIS OF THE QUALITY OF SAMPLES OF PROCESSED FISH MEAT BASED ON THE EXTERNAL APPEARANCE THEREOF, SAID SAMPLES TRAVELLING ON A CONVEYOR BELT SUCH THAT SURFACE DEFECTS CAN BE DETECTED AND THE FISH MEAT CAN BE SORTED ACCORDING TO QUALITY STANDARDS

(57) The invention relates to a method and system for the real-time automatic analysis of the quality of samples of processed fish meat based on the external appearance thereof, said samples travelling on a conveyor belt such that surface defects can be detected and the fish meat can be sorted according to quality standards. The invention comprises the following steps: automatically detecting the processed fish meat samples on the conveyor belt using a tracking method; capturing periodic images of the conveyor belt in order to produce a segmentation of the captured images; analysing the captured image in order to determine the shape and colour of the processed fish meat samples; counting the processed fish meat samples; and detecting surface defects on the processed fish meat samples; and detecting surface defects on the processed fish meat samples, such as haematomas and melanosis. The system comprises; a portable device which is positioned above a conveyor belt and which includes a closed dome-type cover insulated from the surrounding environment, positioned above the conveyor belt, and an L-shaped base structure that is used to position the cover above the conveyor belt without handling same; means for storing and processing information; image capture means; graphical user interface.

FIGURE 1

**Description**

APPLICATION FIELD

The present invention relates to a method and system based on computer vision and pattern recognition techniques for detecting, measuring and analyzing processed fish meats automatically, especially salmon circulating through a conveyor belt, specifically real-time measuring of geometry and color of meat, as well as counting the number of processed pieces for detecting surface defects such as bruises and classifying meats according to patterns of quality.

DESCRIPTION OF PRIOR ART

The use of digital vision for determining food quality has been a subject of active research during the past 30 years, due to progress on computer science and the development of new technologies and methods. Already in the 1970s, it is possible to find research related to the use of computer vision for the analysis of grain quality. In the following years were systematically published works on recognition of features such as form and defects in a variety of foods, fruits, vegetables, grains, fish, meat and manufactured products.

With the massification of digital sensors such as CCD cameras and computational advances, the number and complexity of developments has increased. Nowadays, you can find works that performs automatically real-time analysis of food, performing tasks such as measurement of color, classification and detection of defects, among other applications.

The image processing generally considers five steps:

1) Image acquisition, which may be in analogous format then transforming it to digital format, or directly in digital format with digital cameras;

2) Preprocessing, to obtain an improved image but of the same size that of the original image;

3) Segmentation, in which is the image is partitioned into disjoint and nonoverlapping regions;

4) Measurement of objects, e.g., size, shape, color and texture of image; and

5) Classification for identifying and classifying objects in different groups.

The acquisition using CCD cameras can increase processing speed and also allows obtaining images of higher resolution than the old analogous images. Currently, the most common are those that acquire an image in shades of gray or color, using three components (usually in the UV or RGB (red, Green, Blue) color space).

The preprocessing eliminates the distortion suffered during image acquisition, and is carried out in two possible ways: pixel-level or by neighborhood pixels, depending on the size of the pixel's neighborhood which is used for the calculation of a new pixel. At this step usually is also done a transformation of the RGB color space into HSI (Hue, Saturation, Intensity) or L*a*b* (brightness, red or green content, yellow or blue content). Also, at this step you can compensate for the illumination and make image changes to correct distortions.

Four alternative methods are generally used for image segmentation into their constituent objects: threshold-based, region-based, gradient-based and classification-based. Each method has its own advantages; for example, threshold-based segmentation is particularly effective in scenes containing solid objects that contrast with the background.

The measurement of objects is performed using different attributes to assess quality. In size, for example, area, perimeter, length and width are considered. In form, are included attributes such as area ratio (Area /(Maximum Diameter X Minimum Diameter)), aspect ratio (Maximum Diameter/Minimum Diameter) and circularity (perimeter$^2$/Area). The color is an essential attribute with a high spatial resolution, therefore, it has been used in many foods, including fruit, grain, meat, and vegetables. In terms of texture, it generally quantifies the variations of gray level in a region.

In the scientific field also have been developed methods for the detection of defects in fish fillets, such as, using two simple digital vision algorithms for melanin spot detection , using color information for determining quality in trout; in addition, we have general classification systems such as in the white shrimp case for detecting defects, strange objects, color, and melanosis.

UMEZAWA TADASHI, "Device for inspecting printed wiring boards at different resolutions" US patent 5,784,484 (07/21/1998), describes a device for inspection of printed wiring boards, which measures changes in forms and texture, as well as imperfections given by forms outside of a pre-established pattern of comparison and color changes. However, the present invention is not based on comparisons with a pre-established pattern, but it directly measures different visual variables and recognizes surface defects patterns on fish meats on the basis of a training method based on pattern recognition techniques.

ALLAN ALASIAIR *et all*., "Fish color grader", EP patent 0221642A2 (05/13/1987), describes an apparatus based on the analysis of visible light absorption at different frequencies where the hemoglobin displays a peculiar absorption level. Through the detection of hemoglobin levels it is possible to obtain information about the degree of deterioration of fish flesh. However, the present invention is based on a different technology, computer vision, which allows a non punctual spatial analysis of the information emitted across the visible spectrum, with lighting means throughout the frequency range of this. In addition, thanks to the two-dimensional information received by a video camera, it is possible detecting the position of meats of fish and then analyzes the entire surface, detecting color, form and imperfections patterns such as spots or bruises, in real-time.

NAKAMURA TAKASHI, "DEVICE FOR QUALITY INSPECTION BY IMAGE", JP patent 2001755854 (06/29/2001), describes a device based on image processing techniques which inspect the correct printing of labels and legends of food products. For the operation it is indicated that the system has a master image with the desired characteristics of printing, which is compared with images of products under review. As a result of this comparison, the system is able to detect faults. However, the present proposed invention does not use comparison with pre-established patterns of images, but rather directly measures different variables associated with visual appearance and recognizes patterns of surface defects of fish meats on the basis of a training method based on pattern recognition techniques.

HARTMANN FRANZ and MATERN KLAUS "Apparatus for handling fish fillets for the purpose of quality inspection", US patent 4,706,336 (11/17/1987), describes an apparatus for the visual inspection of fish fillets for the purpose of quality control. The system operates in connection with a conveyor belt that in its central part has the ability to rotate the fillets to carry out the inspection on both sides by a human operator. However, the present invention proposes automatic inspection without human supervision by using computer vision techniques and pattern recognition.

REIMER ERNEST M., "Detection of anomalies in translucent material by candling", US patent 4,978,225 (12/18/1990), describes a inspection system for fish fillets, mainly for the detection of parasites, and it is based on illumination with light beams of different radii, so it uses the relationship between defects and light beam radius. However, the present invention proposes inspection using computer vision techniques and pattern recognition.

REIMER ERNEST M *et all*., "Apparatus and method for automated visual inspection of objects", US patent 6,061,086 (05/09/2000), describes a visual inspection system of products on conveyor belts for the purpose of quality control and is aimed for detecting parasites in fish fillets. This system consists of two cameras; wherein the first one has a viewing angle of low resolution for the first detection of a possible defect, while the second camera has a restricted viewing angle of high resolution to carry out an exhaustive detection of detection indicated by the first camera. However, the present invention is directed to measuring other variables associated with the visual quality of fish meats using apparatus and methods other than those presented in the aforementioned patent.

UMEZAWA TADASHI, "Device for inspecting printed wiring boards at different resolutions", US patent 5,784,484 (07/21/1998), describes a visual inspection system for detecting patterns, in particular visual patterns of printed wiring boards, which acquires a pre-established pattern of the normal appearance of the type of board under inspection, then the pattern model is compared to each board under inspection to detect differences, which indicate some kind of anomaly or fault. However, the present proposed invention does not use image comparison with pre-established patterns, but directly measured different visual variables and recognizes surface defects patterns of fish meats on the basis of a training method based on pattern recognition techniques.

The Canadian company Dipix Technologies (www.dipix.com) specializes in visual inspection systems for food industry, in particular for the determination of color, shape and size of bread, crackers, pizza and tortillas, with more than 150 systems installed in Europe, Asia and Australia. Their inspection systems incorporate various functionalities grouped in three main tools: Qualivision for analysis and processing of data, Q-Reporter, and Envision for generation of reports, charts, and tables.

Integrated Vision Products (www.sickivp.se), a Swedish company, their developments are based on two technologies: a Smart Vision Sensor with 512 processing elements in SIMD architecture and a laser-based linear triangulation system, allowing generating three-dimensional images. Applications include shape and color determination in bottles, wood and paper products, and food products, particularly in cookies.

Baader-Canpolar Inc. (www.baader-canpolar.com) is the developer of Baader-820 Inspektor, a system that automatically separates fish fillets with defects, using a smart camera, advanced image processing software, communication software and mechanical separators arms that removed fillets with defects out of the line, with a processing speed of 6 fillets per second. The detected defects include white membranes, bruising and blood stains.

Marel (www.marel.com), the largest Norwegian company worldwide providing technology for fish and meat processing industry, has three equipments using digital vision. One is QVision, which consists of geometrical measurement system of fillets out of line (sampling). Measuring long, wide, average high and estimating the weight sample from the volume. Other equipment using digital vision is Intelligent Trimming Machine (ITM), which automatically performs the fillets trimming from an image. Baader has an equipment of similar characteristics (www.baader.com). The latter is Intelligent Portioning Machine (IPM), which from an image or a profile obtained with laser portioning fish fillets. A similar function performs the automatic fish fillet cutting system developed by JLI Vision (www.jli.dk), a Danish firm.

Another organization dedicated to technological development in the area processes, is Sintef (www.sintef.no), also from Norway, which has a long history of research and development.

SUMMARY OF THE INVENTION

The present invention provides a method and a system based on computer vision and pattern recognition techniques for detecting, measuring and analyzing processed fish meats automatically, especially salmon circulating through a conveyor belt, specifically real-time measuring of geometry and color of meat, as well as counting the number of processed pieces for detecting surface defects such as bruises and classifying meats according to patterns of quality.

So it provides a method for real-time automated analysis of the quality of processed fish meat samples, based on their external appearance, circulating through a conveyor belt that allows detecting surface defects and classifying the meat according to quality patterns, comprising: detecting automatically the processed fish meat samples on the conveyor belt through a tracking method; capturing periodical images of the conveyor belt in order to perform a segmentation of the captured images; analyzing the captured image to determine the geometry and color of the processed fish meat samples; counting the processed fish meat samples; and detecting surface defects in the processed fish meat samples such as bruising and melanosis.

Similarly, is provided a system for real-time automated analysis of the quality of processed fish meat samples, based on their external appearance, circulating through a conveyor belt that allows detecting surface defects and classifying the meat according to quality patterns, comprising: a portable device that is placed over a conveyor belt transporting fish meats processed samples, comprising: a dome type cover closed and isolated from the surrounding environment, located above the conveyor belt and a "L-shaped" base structure allowing positioning the cover above a conveyor belt without intervention in it; storage and data processing means for: detecting automatically the processed fish meat samples on the conveyor belt by a tracking method; analyzing the captured image to determine the geometry and color of the processed fish meat samples; counting the processed fish meat samples; and detecting surface defects in the processed fish meat samples such as bruising and melanosis; images capture means for capturing periodical images of the conveyor belt; graphical user interface means for accessing predefined parameters and display results; and illumination means to maximize the uniformity of the intensity of light received by processed fish meat samples throughout the space of the image to capture.

BRIEF DESCRIPTION OF THE FIGURES

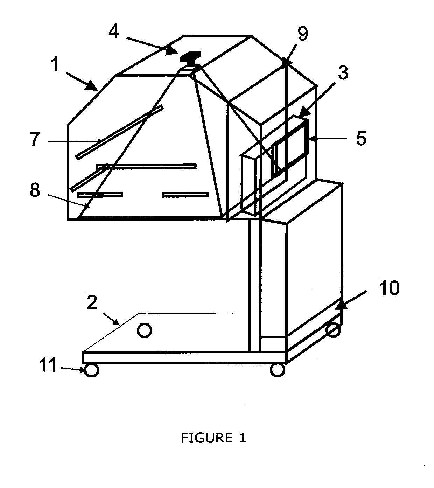

Figure 1 shows the portable unit comprising the system of the present invention.

Figure 2 shows a flow chart diagram of the operation process of the system of the present invention.

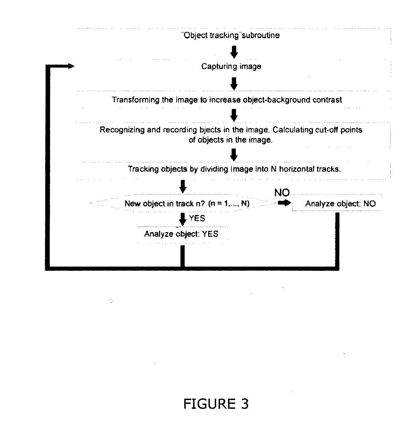

Figure 3 shows the process of the method of tracking objects on a conveyor belt that begins with image capturing.

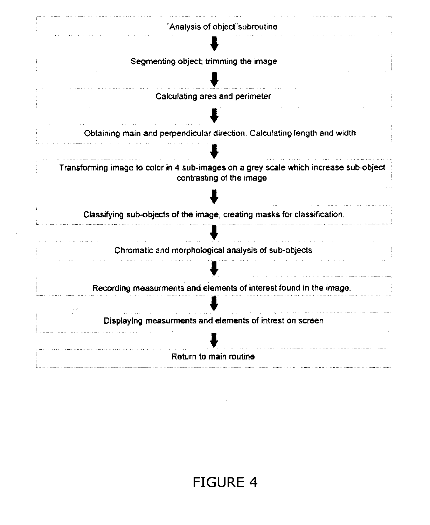

Figure 4 shows the process of analysis of the object captured image on a conveyor belt.

DETAILED DESCRIPTION OF THE INTENTION

The objectives of the present invention relate to provide a method and a system for the real-time analysis of fish meats circulating through a conveyor belt, in particular salmon and trout meat, for determining the quality of these based on their external appearance, which must be operated automatically with and minimal error, in order to be able to replace the work of a human operator.

The presented invention achieves the above mentioned objectives through the development of: i) a system comprising a portable device which is placed above the conveyor belt and using a illumination system, which enabling capturing images of fish meat passing through the conveyor belt selectively; ii) a method based on computer vision techniques and patterns recognition that achieves characterizing the quality of the fish meats based on: automatic detection of processed fish meat samples on the conveyor belt; real-time measuring of the geometry and color of the processed fish meat samples; the counting of processed fish meat samples; and the detection of surface defects such as bruises and melanosis in processed fish meat samples.

The portable device comprising the system of the present invention allows its location above a conveyor belt that transports processed fish meat samples, without the intervention in the conveyor belt structure. This device is preferably made of corrosion-resistant stainless steel and comprises a dome like cover (1) closed and isolated from the surrounding environment, located above the conveyor. To support this cover the device has a structure basis, preferably of stainless steel (2), which also allows positioning the cover above a conveyor belt without the intervention of it. Inside the cover (1), a cabinet is located where are the computer and electrical system (3), separated from the vision section, where the image capture device is installed (4) at the top of the cover (1). Outside of the cover (1) it is placed the user interface screen (5), which serving both to configure the analysis as for display real-time results. Also, inside the cover (1) are the components of the illumination system, corresponding to stable light sources (6) and a light diffuser (7) to obtain stable and uniform illumination in the area of image capturing, which would be the conveyor belt.

To isolate the computer system and electric circuits (8) a steel plate is used to form two independent areas inside the cover (1). The equipment, also, has a counterweight (9) for balancing the equipment, and has casters (10) for easy movement.

The illumination system of the present invention was specially designed to maximize fidelity in the acquisition of colors. This system consists of an array of color fluorescent tubes of 20W (4) and 8W (8), positioned to maximize the uniformity of light intensity that is received by processed fish meat samples, hereinafter the samples, analyzed across the image space. The configuration of the illumination system was designed using a illumination simulation software created for the equipment, wherein is modeled how the image zone is illuminated from the light sources used, assuming that tubes act as point sources of light sorted so that they simulate the existence of these arranged in lines of the size of the tubes and in the same configuration that they would have on the equipment. To remove undesirable reflections, between the light source and the samples, is available a pyramidal shaped light diffuser made of high-density acrylic. The fluorescent tubes are supplied with electronic ballasts of 25 kHz, each one powered by 310V DC power supply. Thus, since the light integration time in the CCD of the camera equipment is on average 8 ms, it ensures a maximum error of 0.5% in the color of successive images, being 0.25% the average error. As the intensity of light is directly dependant on the supply voltage, the equipment has a voltage regulator to deliver a supply voltage for uniform illumination.

In practice, the lighting of the corners is a 90% of the lighting in the centre of the image, so the intensity in the zones distant from the centre of the image is corrected by the software, for obtaining images with uniform illumination. To do this, an image of a homogeneous opaque gray sheet was captured with the equipment. With the captured image a three dimensional matrix is generated, where each element of this matrix is multiplied by the corresponding element of the image (pixel) to obtain a resulting image of homogeneous color, compensating for variations in brightness at the edges. The centre of the image, where the intensity has a maximum, is taken as a reference.

In general, fluorescent tubes deteriorate with usage, giving a different light level as time progresses. Therefore, the illumination must be calibrated for its optimum performance, for which a color palette is provided, divided into 16 boxes. Black and white values are located in the upper boxes, and so are red, green, orange blue, yellow and pink colors, while in the lower boxes are different levels of gray from white to black. To calibrate the equipment the color calibration program is accessed from the configuration, the color palette is positioned over the conveyor belt, and the system detects it and modifies the configuration for capturing images to match the image of the palette colors previously uploaded into the routine.

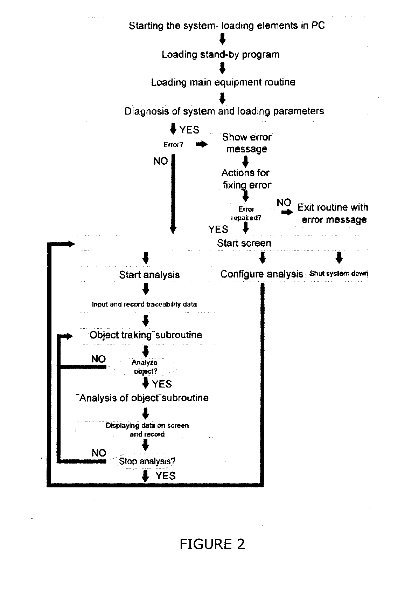

With the operating system, and the conveyor belt loaded with salmon or trout samples and under normal operation the system runs without interruption a tracking method for samples on the conveyor belt, which is schematized in Figure 2, during running, periodic conveyor belt images are obtained in order to make a quick segmentation of the captured images. This segmentation consists of: capturing a high resolution image (1024 x 960 pixels image) of the conveyor belt and samples (fillets) that are on the conveyor belt, thus generating a captured image; resampling the high resolution captured image to obtain an image of smaller dimensions to ensure that processing is fast, generating a resampled captured image; pre-processing the resampled captured image using a linear transformation of the RGB channels to increase the contrast between samples (fillets) and background (conveyor belt), giving an image of high contrast in shades of gray, *i.e.*, greater than a 5% of the change regarding the dynamic range of color components or gray levels, which seeks to assign a value close to one hundred for the pixels belonging to the sample and close to zero for those who do not belong, generating a preprocessed resampled image; thresholding-based segmentation of the preprocessed resampled image, which produces a binary image where the samples appear with a value (usually "one") and the background with another value (generally "zero"), denominated binary resampled image); deleting regions where the samples appear partially outside the binary resampled image, *i.e.*, those where the region touches the edge of the image or stay outside a defined region of interest that is smaller than the image, generating a resampled segmented image. While there are no complete samples (fillets) on the conveyor belt, the resampled segmented image will have only one value in all its pixels (generally "zero").

An algorithm where an image of dimensions W*H is transformed into a new image of dimensions w*h, where W/w = H/h, where the RGB color of each pixel in the resampled captured image is a linear combination of RGB colors in a pixels region of the captured image, is used to generate the resampled captured image. These conditions are given by the camera resolution, where W is the number of horizontal pixels and H the number of vertical pixels of the captured image. For example, if W/w has a value of 4, for each pixel color in the resampled captured image corresponds a RGB color formed by the weighted average number of regions of 4x4 pixels of the captured image.

To generate the preprocessed resampled image, it is required a prior step of parameters configuration for generating two lists; on one hand you have a list of RGB colors of the samples, for example salmon fillets, which is part of the internal data of the equipment, involving all types of tissues that make up the fillet, and on the other hand, a list of RGB colors of the conveyor belt, that is generated at the time of recognition of the conveyor belt, which is part of the equipment configuration options before carrying out the analysis under normal operation. The size of the list of RGB colors of the conveyor belt is similar to the amount of RGB data from the samples, and is made by sampling RGB colors from pixels of the image of the conveyor belt without samples on it. From these two sources of data, it comes obtaining a high contrast sub-image by using a linear transformation of the RGB components that assigns the value of 100 to the colors that make up the sample and - 100 to the colors of the conveyor belt. Once the transformation function by least squares optimization is calculated, high contrast sub-image of the sample-conveyor belt is obtained by applying the transformation to the RGB values of the pixels in the resampled captured image. For the success of this transformation the conveyor belt must have a contrasting color against the samples.

For locating the samples in the image, the tracking method of the invention uses the segmentation described in the preceding paragraphs, and also uses a monitoring system for the found samples in the actual resampled segmented images based on samples found in the previous resampled segmented images, which comprises a position vector (x, y) of the centres of area from the regions of the resampled segmented image, where "x" is the value of the horizontal coordinate measured from left to right in the image, and "y" is the value of vertical coordinate measured from top to bottom in the image; a virtual horizontal tracks created and destroyed dynamically (horizontal tracks); a vertical position vector of the horizontal tracks centres; and horizontal position vectors for monitoring the samples of each track .

The movement of the conveyor belt and the position of the digital camera in the equipment produce samples to move horizontally in the image, for example, from left to right. The camera is the only sensor in the equipment, in this case the image sensor, which is capable of obtaining tens of high resolution images per second. In successive images, a sample that advances through the conveyor belt varies its position horizontally in the image.

The image that is used for tracking corresponds to the resampled segmented image, so in successive images only the regions corresponding to complete samples contained in the captured image are seen move forward horizontally (or in a region of interest of this), so the vertical position "y" of the centre of area of these regions will have slight variations, while that variation will be mainly in the horizontal coordinate "x" of the centre of area. If the movement is from left to right, in each successive image the "x" coordinate of the centre of area of a particular region increases.

The number of tracks at the starting of normal operation is zero. At the time of appearing the first sample in the captured image, this sample appears as a region in the resampled segmented image only when it is completely contained in the captured image. At the time that the region appears on the resampled segmented image, the centre of area of the region is calculated, which is stored in the position vector (x, y) of the centres of area. As it is the first region found, and there are no tracks created, the first horizontal track is created, corresponding to a rectangular region of horizontal length equal to the horizontal length of the resampled segmented image, and a vertical length preferably of 8 pixels, whose centre is the coordinate "y" of the centre of area calculated in the found region. The value "y" is stored on the first box of the vertical position vector of centres of horizontal tracks, and the "x" value is stored on the first box of the horizontal position vector for monitoring samples. There are no more operations, so the system acquires another image after making these processes.

At the time of acquiring the next captured image and generate the corresponding resampled segmented image, the movement of the conveyor belt produces the horizontal movement in a few pixels of the region previously identified in the new image. In this case, the system checks to see if the region corresponds to the same found in the previous image, by comparing the coordinates of the position of the centre of area of this region with the values stored in the horizontal and vertical position vectors of the tracks. Being in the same region but moved horizontally, and also counting with only one track created, the "y" value of the centre of area of the region is contained within the created track, while the "x" value of the centre of area of the region is greater than the value stored in the horizontal position vector for monitoring. Therefore, in this case, the first entry of the horizontal position vector for monitoring is updated with the new "x" value, while the first box of the vertical position vector of centres of tracks remains unchanged since it is the same track.

In another case, a second region can appear in the resampled segmented image, corresponding to a second sample. In this case, the same procedure is done. For the first region, the procedure is the same as in the previous paragraph. For the second, if it does not belong to the track created with the first found region, it is created a new track using the same procedure as for the first found region and storing coordinates "x" and "y" of the centre of area of this new region in the corresponding vectors, in the second box, so in this case the number of tracks are two. On the contrary, if it belongs to the same track, and the "x" value of its centre of area is smaller than the value stored in the first box in the horizontal position vector for monitoring, the value of the first entry is conserved without updating.

The process of creating horizontal tracks continues for other regions appearing in successive images. Every time a region appears, and when the "y" value of its centre of area do not belong to any track, a new track will be created and it will be monitored as indicated above. One of the virtues of the dynamic creation of tracks is that makes unnecessary to use other sensors that detect the presence and position of samples on the conveyor belt, in the same way; its generality avoids the need for a specific order of appearance of the samples on the conveyor belt to follow his horizontal movement.

Once the tracks have been assigned or updated for all the regions of the resampled segmented image, a search (scanning) in the horizontal position vector is performed for monitoring each track looking if an "x" value crosses the centre of resampled segmented image, *i.e.* when the "x" value of the above mentioned vector is greater than w/2. If this is true for some track, the original captured image and the coordinates of the boundaries of the region corresponding to the sample to analyze are introduce into the analysis routine. Using multitasking programming techniques the analysis process of the samples is started in parallel with the tracking process. In this case, if there is another(s) sample(s) that must be analyzed(s), before acquiring a new image a complete scanning is performed again by the horizontal position vector for monitoring tracks. If there are no more samples to analyze, a new image is captured and the track corresponding to the analyzed sample is erased, so it can be occupied by another object. If another sample should be analyzed, the analysis is performed in parallel when finished the sample that was analyzed previously. This process is repeated until there are no more samples to be analyzed and a new image can be captured. Through this system, several samples can be positioned in parallel on the conveyor belt, and the system will be able to analyze them individually.

If tracks overlap, the "y" value of the coordinate of the centre of area might belong to both tracks, in which case the region is assigned to the track whose "y" value of the centre track is closest to the "y" value of the centre of area of the region; in the case that the "y" value of the coordinate of the centre of area of a new region is too close (less than 2 pixels) of the vertical limit (top or bottom) of a track, and that it belongs to this, a new horizontal track is created, which is centered in the "y" value of the coordinate of the object centre of area.

Regions in the resampled binary image that have already crossed the half of this image but are also contained completely within the image are also removed from the resampled segmented image. To avoid problems updating a track when a region disappears, the values of the position vectors corresponding to the boxes of the existing tracks, but without the resampled segmented image regions within them, are artificially updated, *i.e.*, its corresponding value in the horizontal position vector for monitoring is increased.

In terms of the analysis process of each detected sample, the first thing is to receive the original captured image and the coordinates of the boundaries where the object is located to analyze. Then, an analogous segmentation to the one already carried out is performed, getting the resampled image during the tracking process. This analysis is performed in the original captured image, without dimensionality reduction, in order to give greater detail and thus greater accuracy measurements.

After the initial segmentation for separating subject and background, the measurement of geometric features such as area, perimeter, length, and width is performed. The total count of pixels that make up the object corresponds to the area, while for the perimeter the pixels of the contour of the object are counted.

To calculate the length and width of the samples, a rectangle is circumscribed to said sample, identifying the sample length with the rectangle greater length and the sample width with the rectangle smaller length.

Then, it proceeds to separation of elements of the same object. This separation is done through a transformation whose input are the three channels of the original image (R, G and B) and whose output are images of intensity or shades of gray, oriented to form high-contrast of a image element in particular; therefore, there will be as many images as elements wanted for discrimination. This transformation parameters corresponds to the optimization of a non-linear function in R, G and B, but lineal in the coefficients involving components i, i*j y $\sqrt{i}$, where i, j = R,G,B.

Thus, to find the parameters became available a training set consisting of four lists of RGB colors corresponding to the four tissues that are identified in the images (muscle, peritoneum/white fat, brown fat, and hematoma/melanosis). Each element of these lists has three components, corresponding to the values of R, G and B of the corresponding color for the pixel. To generate these lists a training set of 50 typical sample images, in this case of salmon fillets, which include healthy fillets with and without white fat (peritoneum), with and without the presence of defects and stains, and with and without brown fat, were available. The method requires at least 30 typical images to be carried out and have satisfactory results.

First, tissues are separated manually and color and form data of these are obtained. With the use of these images, regions are manually selected corresponding to each tissue, each region with an identifier of the corresponding tissue, and using these regions is generated a lists of RGB color. In addition, for images with defects was stored, in a separate list, geometric information of the defect, such as size, shape and location. Later, by means of linear least squares optimization is seeks to find a linear transformation of parameters (quadratic in the RGB variables), such that for the triads of RGB colors of a particular tissues (e.g., muscle) is assigned a value of 100 for the RGB colors associated with tissue, and - 100 to other tissues. Practically, it had four transformations, one for each tissue that is wanted to identify. Generally, with n elements sought in the image, a typical transformation would be for a pixel with R, G, and B color values:

$$b = a_1 R + a_2 G + a_3 B + a_4 R^2 + a_5 G^2 + a_6 B^2 + a_7 RG + a_8 RB + a_9 GB + a_{10}\sqrt{R} + a_{11}\sqrt{G} + a_{12}\sqrt{B}$$

The $a_k$ coefficients, k = 1,..., 12, are calculated on the basis of a least squares estimation , where the b value is assigned as 100 or - 100 if the RGB color of the pixel corresponds to the element designated or not, respectively. The $a_k$ coefficients can be grouped into a $M_{Kxn}$ matrix of *n* elements, which correspond to the four tissues in the case of the invention.

Once done the training, the $a_k$ coefficients are fixed parameters of the subroutine's analysis of objects, which are loaded when you start the system.

With the sub-images created with this technique, it is possible analyzing elements or tissues of the image separately, where *n* sub-images of one channel, similar to gray intensity images, are generated from a RGB color image.

For the specific salmon case, four sub-images are generated: muscle, white fat and brown fat and hematoma. The sub-images correspond to grey intensity images (one intensity channel). For obtaining the regions corresponding to each tissue, those sub-images are carried out to the threshold. Since the images have a range approximately between - 100 and 100, it was found that the optimal thresholds for each tissue is 10 for muscle, -26 for peritoneum/white fat, -20 for brown fat and -16 for hematoma/melanosis. With these thresholds by themselves is not possible obtaining instantly and directly the regions corresponding to each tissue in particular, since tissues are usually not completely homogeneous and there are pixels in sub-images that give values that do not correspond to the desired tissue, for which there is an intermediate processing for seeking clean regions, so there is no overlap between them, and for deleting those pixels where the classification is not clear or is inaccurate. For this reason, cross information of the regions is used to prevent overlapped areas in the regions corresponding to tissues, and then through morphological analysis of the regions is possible deleting sectors of regions which are very small (in the order of few pixels) or because the characteristics of the samples of the found region does not belong to the place where must typically be, such as bruising on the tip of the fillet, elongated shaped bruises, white fat found that indeed correspond to muscle myoceptum that are removed due to its elongated shape, among others. Once the regions have been identified masks are generated that will be used for the measurement and classification of the fillet, corresponding to binary images, 0 and I values, so that a 0 value indicates that the pixel does not correspond to the tissue and 1 indicates that it does. In particular, the fillet is subjected to chromatic and geometrical measurements, overlapping each mask to the original image.

The system is capable of detecting bruising and melanosis in the salmon fillet which area is greater than 0.5 $cm^2$. The detection of defects is based on the creation of models of detection or classifiers, from at least 30 typical images, preferably a set of 50 typical images of samples is used as a set for training, where 25 of these correspond to samples with defects (hematoma/melaniosis). For recognizing spots, bruises and melanosis, is first obtained the segmentation of these, as explained in the previous paragraph. Once the defect is isolated, we proceed to evaluate whether the defect found is true or false, to which classifiers constructed on the basis of measurements obtained in the training set, are used. For example, in the case of melanosis, the training set allows to obtain probability distributions over: minimum and maximum sizes, typical location within the fillet, and comparison of the defect with the surrounding tissue, among others. Then, these distributions are used to classify if a potential visual anomaly of the fillet is actually a defect or not. In this sense the detection focuses on a system of pattern recognition, where there are the parameters that define the faults, as opposed to directly compare the fillet with one of sample as it would be the case using a template for comparison or a typical image of salmon and compare it directly with the captured image. The equipment does not use direct comparative techniques since the samples, salmon fillets, show great variability, unlike the case of analyzing products equal to each other (e.g. printed wired boards) where a comparison template can be use. Due to the variability of the samples, above mentioned distributions give general information about the morphology and location of the defect, which is why each possible defect found is verified through these distributions to verify if it is within normal parameters or "pattern".

For other tissues (muscle, white fat/peritoneum and brown fat) the average color and geometry are measured using the captured image and the information of the generated masks.

In terms of color measurement is used as unit of measurement the industrial standard "SalmoFan" developed by Hoffmann-LaRoche. The measurement covers the full range of the coloration of salmon muscle on a scale of 20 to 34. This measurement is performed considering only the color of the muscle between the 25 and 75 percentiles to remove data out of range, since there are areas in the salmon that are naturally very dark or very clear, they do not influence on the decision of an expert in relation to the coloration. The color is then measured and gets the component L* of the color average measured by a non linear transformation from RGB color described in Mery, 2006, K. León, D. Mery, F. Pedreschi, J. León (2006) "Color measurement in L*a*b* units de RGB digital images". Food Research International, 39, 1084-1091.

The L* component is the one that most resembles the valuation of the color measured by experts. At this step the equipment measurements in the "SalmonFan" scale are calibrated according to expert human operators in order to match the measurements of the equipment and the experts.

For displaying of the results on-line are used time graphics, histograms and measured value; all displayed on the screen according to the configuration of the user. Graphics are shown as samples are analyzed, and its scale is automatically adjusted to values that are given from the analysis.

Another way of displaying results is through a Web interface. In this case histograms and graphs are obtained at the time of the previous analyses (historical). Mainly, the access to this Web interface is from a terminal connected to the internal Internet of the company or plant. In addition, through a system of authorized accounts, it is also possible to configure the connection from any remote terminal with internet access.

A characteristic of the system and method is that it identifies all objects analyzed, for which it counts the objects that pass through the conveyor belt, so it can be used as a counter for objects that pass through the conveyor belt.

**Claims**

1. A method for real-time automated analysis of the quality of processed fish meats samples based on their external appearance, circulating through a conveyor belt allowing the detection of surface defects and classification of meats according to quality patterns, **CHARACTERIZED by** comprising the steps of:

    a. automatically detecting the processed fish meat samples on the conveyor belt by a tracking method;
    b. capturing periodic images of the conveyor belt in order carry out a captured images segmentation; and
    c. analyzing the captured image to determine the geometry and color of processed fish meat samples; counting the processed fish meat samples; and detecting surface defects in the processed fish meat samples, such as bruising and melanosis;

    wherein, in the analyzing step the capture of the image comprises carry out a segmentation to get a resampled image during the tracking method, for separating the processed fish meat sample and the conveyor belt.

2. The method according to claim 1, CHARACTERIZED because said captured images segmentation comprising:

    a. capturing an high resolution image (1024 x 960 pixels) of the conveyor belt and processing fish meat samples existing on the conveyor belt;
    b. resampling the high resolution captured image to obtain an image of smaller dimensions to ensure that processing is fast;
    c. preprocessing the resampled captured image using a linear transformation of the RGB channels (Red, Green, Blue) to increase the contrast between the processed fish meat samples and the conveyor belt, giving an image of high contrast in shades of gray, *i.e.* greater than a 5% of change regarding to the dynamic range of color components or gray levels, which seeks to assign a value close to a hundred for pixels belonging to the processed fish meat samples and close to zero for those who do not belong;
    d. segmenting by thresholding the preprocessed resampled image, which produces a binary image where processed fish meat samples appear with a value, generally "one", and the conveyor belt with another value, generally "zero";
    e. deleting regions where the samples appear partially outside of the binary resampled image, i.e., those where the region touches the edge of the image or stay outside from a defined region of interest that is smaller than the image.

3. The method according to claim 2, CHARACTERIZED because for resampling the captured image uses an image of dimensions WxH which is transformed into a new image with dimensions wxh, where is true that W/w = H/h, and where the RGB color of each pixel from the resampled captured image is a linear combination of RGB colors of a pixels region from the captured image, where W is the number of horizontal pixels and H is the number of vertical pixels of the captured image.

4. The method according to claim 2, CHARACTERIZED because preprocessing the resampled image requires a prior step of configuration of parameters for the generation of two lists.

5. The method according to claim 4, CHARACTERIZED because for the generation of the two lists is provided, for the first list, a database comprising a list of RGB colors of fish meat samples that identifies the types of tissue comprising said processed fish meat sample; and for the second list, a database comprising a list of RGB colors from the conveyor belt.

6. The method according to claim 5, CHARACTERIZED because with these two lists a high contrast sub-image is obtained by using a linear transformation of the RGB components that assign a value of 100 to the colors comprising the processed fish meat sample, and - 100 to the colors of the conveyor belt.

7. The method according to claim 2, CHARACTERIZED because the tracking method uses said segmentation of the captured images and the monitoring of the samples found in the actual resampled segmented image based on samples found in the previous resampled segmented images, which comprises:

   a. a position vector (x, y) of centres of area in the regions of the resampled segmented image, where "x" is the horizontal coordinate value measured from left to right in the captured image, and "y" is the vertical coordinate value measured from top to bottom in the captured image;
   b. a virtual horizontal tracks created and destroyed dynamically (horizontal tracks);
   c. a vertical position vector of the horizontal tracks centres; and
   d. horizontal position vectors for monitoring samples in each horizontal track.

8. The method according to claim 7, CHARACTERIZED because the number of horizontal tracks at the start of the process is zero.

9. The method according to claim 8, CHARACTERIZED because when appears the first processed fish sample in the captured image, it appears as a region in the resampled segmented image only when it is entirely contained in the captured image.

10. The method according to claim 9, CHARACTERIZED because for said region from the resampled segmented image, is calculated the centre of area of the region, which is stored in a position vector (x, y) of the centres of area.

11. The method according to claim 10, CHARACTERIZED because it is created a first horizontal track corresponding to a rectangular region of horizontal length equal to the horizontal length of the resampled segmented image, and vertical length preferably of 8 pixels, which centre will be the "y" coordinate of the centre of area calculated in the found region.

12. The method according to claim 11, CHARACTERIZED because the "y" value is stored on the first box of the vertical position vector of centres of horizontal tracks, and the "x" value, on the first box of the horizontal position vector for monitoring processed fish meat samples.

13. The method according to claim 12, CHARACTERIZED because each time that a region appears in the resampled segmented image and also the "y" value of its centre of area does not belong to any track, will be created a new horizontal track.

14. The method according to claims 3 and 13, CHARACTERIZED because once the horizontal tracks have been assigned or updated for all regions of the resampled segmented image, searching or scanning in the horizontal position vector is performed for monitoring each track looking if an "x" value is greater than w/2, *i.e.*, crosses the centre of the resampled segmented image, i.e. when the "x" value of the above mentioned vector, to start analyzing the captured image.

15. The method according to claim 1, CHARACTERIZED because for analyzing each processed fish meat sample is necessary to have the captured image and the coordinates of the limits where said sample to be analyzed is located.

16. The method according to claim 1, CHARACTERIZED because the geometric characteristics of fish meats samples comprise area, perimeter, length, and width of the processed fish meat samples.

17. The method according to claim 16, CHARACTERIZED because determining the area of the processed fish meat sample comprises making a total count of pixels in said sample.

18. The method according to claim 16, CHARACTERIZED because determining the perimeter of the processed fish meat samples comprises making a counting of pixels in said sample.

19. The method according to claim 16, CHARACTERIZED because determining the length and width of the processed fish meat sample, comprises circumscribing a rectangle to said sample, identifying the sample length with the rectangle greater length and the sample width with the rectangle smaller length.

20. The method according to claim 1, CHARACTERIZED because detecting surface defects in the fish meat samples comprises identifying four types of tissues, corresponding to muscle, peritoneum/white fat, brown fat, and hematoma/melanosis, in said samples.

21. The method according to claim 20, CHARACTERIZED because became available a training set consisting of four lists of RGB colors corresponding to the four tissues that are identified in the captured images, where each element of these lists has three components, corresponding to the values of R, G and B of the corresponding pixel color.

22. The method according to claim 21, CHARACTERIZED because for generating these lists of RGB colors was available said training set with at least 30 typical images ofprocessed fish meat samples.

23. The method according to claim 21, CHARACTERIZED because the training set comprises preferably 50 typical images of processed fish meat samples.

24. The method according to claim 23, CHARACTERIZED because said typical images of processed fish meat samples comprises healthy processed fish meat samples, processed fish meat samples with white fat, processed fish meat samples without white fat (peritoneum), processed fish meat samples with presence of defects and stains, processed fish meat samples without the presence of defects and stains, processed fish meat samples with brown fat and processed fish meat samples without brown fat.

25. The method according to claim 24, CHARACTERIZED because obtaining said typical images of processed fish meat samples comprises:

   a. separating manually the tissues and obtaining color and form data from said tissues;
   b. selecting manually regions corresponding to each tissue in said typical images; and
   c. assigning an identification for each tissue and generating the RGB color lists using these regions.

26. The method according to claim 25, CHARACTERIZED because for identifying said types of tissues is made a mathematical transformation whose input are the three channels of the original image (R, G and B) and whose output are images of intensity or shades of gray, oriented to form high-contrast of a image element in particular; therefore, there will be as many images as elements wanted for discrimination.

27. The method according to claim 26 CHARACTERIZED because said mathematical transformation is a linear transformation of the type:

$$b = a_1 R + a_2 G + a_3 B + a_4 R^2 + a_5 G^2 + a_6 B^2 + a_7 RG + a_8 RB + a_9 GB + a_{10} \sqrt{R} + a_{11} \sqrt{G} + a_{12} \sqrt{B}$$

where $a_k$, k = 1,..., 12, are calculated on the basis of least squares estimation , where the b value is assigned as 100 or - 100 if the RGB color of the pixel corresponds to a particular tissue or not , respectively.

28. The method according to claim 27, CHARACTERIZED because "n" sub-images in gray intensity in a single channel, where "n" is the number of tissues to analyze in the processed fish meat sample, are generated from the RGB color image.

29. The method according to claim 28, CHARACTERIZED because thresholding is applied over said sub-images for obtaining regions corresponding to each tissue in the processed fish meat samples.

30. The method according to claim 29, CHARACTERIZED because said thresholding comprising values for each tissue, which consists of putting a value to muscle tissue of 10, -26 for peritoneum/white fat, -20 for brown fat and - 16 for hematoma/melanosis.

31. The method according to claim 30, CHARACTERIZED because to avoid overlapped areas in the regions corresponding to the tissues is used cross information from said regions, and then by morphological analysis very small sectors of a region are deleted or by the characteristics of the processed fish meat samples, the tissue region found does not belong to the place where must typically be.

32. The method according to claim 31, CHARACTERIZED because once the regions have been identified masks are generated for the measurement and classification of the processed fish meat sample, which correspond to binary images, values 0 and 1, such that a value of 0 indicates that the pixel does not correspond to the tissue and 1 indicates that corresponds to the tissue.

33. The method according to claim 32, CHARACTERIZED because is carried out a superposition of each generated mask and the captured image allowing geometric and color measurements of the processed fish meat sample and by pattern recognition the parameters that define the surface defects in the processed fish meat samples, such as bruises and melanosis, are obtained.

34. A system for real-time automated analysis of the quality of processed fish meats samples based on their external appearance, circulating through a conveyor belt allowing the detection of surface defects and classification of meats according to quality patterns, **CHARACTERIZED by** comprising:

    a. a portable device that is placed over a conveyor belt transporting fish meats processed samples, comprising: a dome type cover closed and isolated from the surrounding environment, located above the conveyor belt and a "L-shaped" base structure allowing positioning the cover above a conveyor belt without intervention in it;
    b. storage and data processing means for: detecting automatically the processed fish meat samples on the conveyor belt by a tracking method; analyzing the captured image to determine the geometry and color of the processed fish meat samples; counting the processed fish meat samples; and detecting surface defects in the processed fish meat samples such as bruising and melanosis;
    c. images capture means for capturing periodical images of the conveyor belt;
    d. graphical user interface means for entering predefined parameters and display results; and
    e. illumination means to maximize the uniformity of the intensity of light received by processed fish meat samples throughout the space of the image to capture.

35. The system according to claim 34, CHARACTERIZED because inside the closed and isolated cover a cabinet is located containing: storage and data processing means; and image capture means.

36. The system according to claim 34, CHARACTERIZED because outside of the closed and isolated cover are located the graphical user interface means.

37. The system according to claim 34, CHARACTERIZED because the illumination means comprises stable light sources and a light diffuser to obtain a stable and uniform illumination in the area of image capture.

38. The system according to claim 37, CHARACTERIZED because the stable light sources correspond to an array of fluorescent tubes positioned to maximize the uniformity of light intensity that is received by processed fish meat samples; wherein, the fluorescent tubes are supplied with electronic ballasts, which in turn are powered by DC power supply, so that a voltage regulator allows to deliver a uniform voltage supply for illumination, regulating light intensity and decreasing color errors of successive images.

39. The system according to claim 34, CHARACTERIZED because the portable device also includes a counterweight to balance the device and casters for moving.

40. The system according to claim 34, CHARACTERIZED because processed fish meat samples are salmon fillets.

41. The system according to claim 34, CHARACTERIZED because processed fish meat samples are trout fillets.

FIGURE 1

Starting the system- loading elements in PC
↓
Loading stand-by program
↓
Loading main equipment routine
↓
Diagnosis of system and loading parameters
↓ YES

Error? ➡ Show error message
↓
NO
Actions for fixing error
↓
Error repaired? NO ➡ Exit routine with error message
YES ↓
Start screen

↓ Start analysis
↓
Input and record traceability data
↓
Object traking subroutine
↓
NO Analyze object?
↓ YES
Analysis of object subroutine
↓
Displaying data on screen and record
↓
NO Stop analysis?
↓ YES

Configure analysis    Shut system down

FIGURE 2

Object tracking subroutine

⬇

Capturing image

⬇

Transforming the image to increase object-background contrast

⬇

Recognizing and recording bjects in the image. Calculating cut-off points of objects in the image.

⬇

Tracking objects by dividing image into N horizontal tracks.

⬇

New object in track n? (n = 1,..., N)   NO ➡ Analyze object: NO

⬇ YES

Analyze object: YES

FIGURE 3

"Analysis of object" subroutine

⬇

Segmenting object; trimming the image

⬇

Calculating area and perimeter

⬇

Obtaining main and perpendicular direction. Calculating length and width

⬇

Transforming image to color in 4 sub-images on a grey scale which increase sub-object contrasting of the image

⬇

Classifying sub-objects of the image, creating masks for classification.

⬇

Chromatic and morphological analysis of sub-objects

⬇

Recording measurments and elements of interest found in the image.

⬇

Displaying measurments and elements of intrest on screen

⬇

Return to main routine

FIGURE 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5784484 A
- EP 0221642 A2
- JP 2001755854 B
- US 4706336 A
- US 4978225 A
- US 6061086 A

**Non-patent literature cited in the description**

- **K. LEÓN ; D. MERY ; F. PEDRESCHI ; J. LEÓN.** Color measurement in L*a*b* units de RGB digital images. *Food Research International,* 2006, vol. 39, 1084-1091